# EUROPÄISCHE PATENTSCHRIFT

(11) **EP 2 240 210 B1**
(45) Veröffentlichungstag und Bekanntmachung des Hinweises auf die Patenterteilung: **11.05.2011**
(21) Anmeldenummer: 08867355.3
(22) Anmeldetag: 17.12.2008
(51) Int. Cl.: A61L 9/16, A61L 9/12, A61L 2/18, A61L 2/22, B65D 90/44, A61L 9/14

(54) **VERFAHREN ZUM REDUZIEREN VON ÜBELRIECHENDEN SUBSTANZEN IN TANKBEHÄLTERN**
METHOD FOR REDUCING MALODOROUS SUBSTANCES IN TANK CONTAINERS
PROCÉDÉ DE RÉDUCTION DE SUBSTANCES MALODORANTES DANS DES RÉSERVOIRS

(30) Priorität: 21.12.2007 DE 102007062314
(43) Veröffentlichungstag der Anmeldung: 20.10.2010
(73) Patentinhaber: Air & D- Sarl, 67190 Rosheim (FR)
(72) Erfinder: WUEST, Robert, F-67190 Rosheim (FR)
(74) Vertreter: Nuss, Laurent
(86) Internationale Anmeldenummer: PCT/EP2008/010766
(87) Internationale Veröffentlichungsnummer: WO 2009/083156

(56) Entgegenhaltungen:
- EP-A- 1 754 493
- EP-A- 1 772 158
- WO-A-01/36354
- WO-A-2004/099352

## Beschreibung

Die Erfindung betrifft ein Verfahren zum Reduzieren von übelriechenden Substanzen in Tankbehältern für Schweröl oder Bitumen, in denen über dem Flüssigkeitsspiegel sich ein Gasraum mit den übelriechenden Substanzen befindet, wobei in den Gasraum aktive Agenzien eingeleitet werden, die mit den übelriechenden Substanzen reagieren oder diese maskieren.

Schweröle und verflüssigtes Bitumen werden im allgemeinen in großen Tankbehältern, die bis zu 40 m hoch sein können, gelagert. Je nach Füllgrad des Tanks befindet sich über dem Flüssigkeitsspiegel ein mehr oder weniger großer Gasraum, in den hinein flüchtige Bestandteile aus der Flüssigkeit verdunsten. In diesen Gasraum werden in manchen Fällen, aber nicht immer, Inertgase eingeleitet, die am oberen Ende des Tankbehälters zusammen mit den flüchtigen Bestandteilen wieder abgeführt werden. Diese flüchtigen Bestandteile bestehen im wesentlichen aus Kohlenwasserstoffen und Schwefelverbindungen, die in der Umgebung des Behälters einen üblen, zum Erbrechen reizenden Geruch verbreiten. Dies ist besonders gravierend, wenn der Tank gefüllt wird und durch den dabei auftretenden erhöhten Druck die Entgasung durch das Abluftrohr beschleunigt wird. Es ist nun praktisch nicht möglich, die übelriechenden Substanzen auf konventionelle Weise durch Absaugen zu entfernen, da einige ihrer Bestandteile in klebriger Form kondensieren und so die Absaugrohre verstopfen würden.

Der Erfindung lag nun die Aufgabe zugrunde, die übelriechenden Substanzen in dem Gasraum zu entfernen oder wenigstens stark zu reduzieren, um dadurch die Geruchsbelästigung zu vermindern.

In der EP-A 1 772 158 ist ein Verfahren zum Reduzieren von übelriechenden Substanzen in Tankbehältern für Schweröl und Bitumen beschrieben, bei dem in den Gasraum über dem Flüssigkeitsspiegel zusammen mit einem Inertgas aktive Agenzien eingeleitet werden, die mit den übelriechenden Substanzen reagieren bzw. diese maskieren und dadurch die Geruchsbelästigung vermindern. Die aktiven Agenzien werden dabei, vorzugsweise als wässrige Emulsion in Form feiner Tröpfchen, in das Inertgas eingepresst und mit diesem zusammen in den Gasraum eingeführt. Dieses recht komplizierte Verfahren sollte verbessert und außerdem auf solche Tankbehälter ausgedehnt werden, bei denen kein Inertgas eingeleitet wird.

WO 2004/099352 umfasst die Eliminierung von Gerüchen in ölhaltigen Substanzen.

WO 01 363 54 lehrt kohlenwasserstoffhaltige Zusammensetzungen geeignet zum Zusatz zu Asphalt und Mitteldestillaten. Diese Zusammensetzungen können Aldehyde oder Ketone enthalten.

Diese Aufgaben werden erfindungsgemäß durch die Merkmale des Anspruchs 1 gelöst.

Im industriellen Bereich werden recht große Tankbehälter verwendet, die z.B. 500 bis 15.000 to Schweröl bzw. Bitumen enthalten. Bitumen liegt dabei als 80 bis 200 °C heiße Flüssigkeit vor, wodurch natürlich die Verdunstung von flüchtigen Bestandteilen begünstigt wird.

Je nach Füll- bzw. Entleerungsgrad des Tankbehälters macht der Gasraum über dem Flüssigkeitsspiegel 10 bis 90 % des Gesamtvolumens aus. In diesen Gasraum hinein kann zur Inertisierung gegen Explosionen ein Inertgas eingeleitet werden, z.B. Stickstoff oder Wasserdampf, wobei letzterer im allgemeinen unter einem Druck von 2 bis 20 bar steht und eine Temperatur von 140 bis 200 °C aufweist. In diesem Fall werden die aktiven Agenzien direkt in den Gasraum eingesprüht und zwar gleichzeitig mit dem Inertgas, jedoch unabhängig von diesem durch eine separate Düse.

Bei der Erfindung werden die aktiven Agenzien in den Gasraum eingesprüht, ohne dass gleichzeitig ein Inertgas eingeleitet wird. Die aktiven Agenzien können seitlich vom Abgasrohr in den Gasraum hinein oder direkt in das Abgasrohr hinein eingesprüht werden.

Erfindungsgemäß enthalten die aktiven Agenzien mindestens 30 Gew.% eines Aldehyds mit mehr als 10 Kohlenstoffatomen im Molekül und einem Siedepunkt von mindestens 150 °C, und sie werden in Mischung mit 20 bis 95, vorzugsweise 30 bis 70 Gew.% eines Verdünnungsmittels eingesetzt. Das Verdünnungsmittel bewirkt, dass die aktiven Agenzien sehr schnell und gleichmäßig auf der Flüssigkeitsoberfläche verteilt werden und so unmittelbar nach dem Einsprühen ihre Wirksamkeit im Gasraum entfalten können.

Als Verdünnungsmittel wird ein Mineralöl eingesetzt, d.h. ein Kohlenwasserstoffmitteldestillat mit einem Siedebereich zwischen 180 und 360 °C.Besonders bevorzugt ist CATENEX von der Fa. SHELL.

Neben den Aldehyden können die aktiven Agenzien noch natürlich vorkommende Alkohole, Terpene, Ketone sowie Carbonsäureester, jeweils mit Siedepunkten oberhalb von 150 °C enthalten.

Eine besonders bevorzugte Mischung von aktiven Agenzien enthält:
A. 30 bis 60 Gew.% eines Aldehyds, z.B. alpha- Hexylcinnamaldehyd, 2-(4-tert.-butylbenzyl)propionaldehyd, 2-Benzylheptanal, Citral, 2-Methylundecanal, 3-(4-Isopropylphenyl)2-methylpropanal oder Mischungen davon,
B. 10 bis 40 Gew.% eines Alkohols, z.B. Linalol, Eugenol, Geraniol, alpha-Terpineol, Vanillin, Thymol oder Mischungen davon,
C. 0 bis 40 Gew.% eines Terpens, z.B. Limonen, alpha-Pinen, Cymol oder Mischungen davon,
D. 0 bis 40 Gew.% Ketone, z.B. Benzophenon, Butylmethylketon, 1,6-Cineol oder Mischungen davon, sowie
E. 0 bis 40 Gew.% Carbonsäureester, z.B. Benzylsalicylat, Amylbutyrat, Ethylbutyrat, Amylacetat oder Mischungen davon. wobei sich die Prozentzahlen auf 100 addieren.

Die in den Gasraum eingeführten aktiven Agenzien reagieren dort mit den übelriechenden Substanzen und entfernen diese ganz oder zumindest teilweise aus dem austretenden Abgasstrom, so dass die Geruchsbelästigung weitgehend beseitigt ist. Gleichzeitig können wohlriechende aktive Agenzien auch die übelriechenden Substanzen maskieren und deren Geruch überdecken.

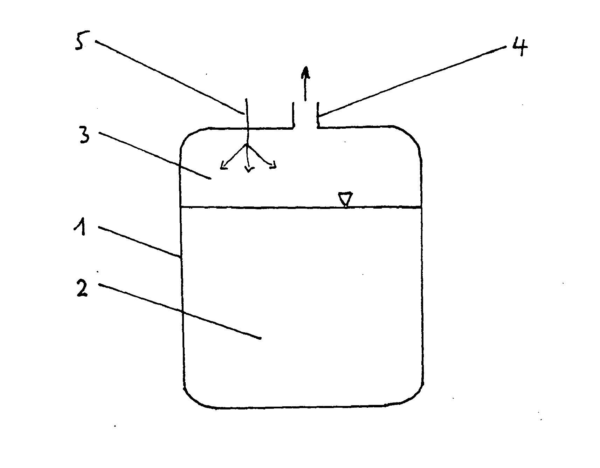

Die beigefügte Zeichnung zeigt eine schematische Verlaufsskizze der bevorzugten Ausführungsform. Dabei ist mit **1** der Tankbehälter bezeichnet, mit **2** das Schweröl bzw. das verflüssigte Bitumen, mit **3** der Gasraum über dem Flüssigkeitsspiegel, mit **4** der Austritt des Abgases, mit **5** die Düse, durch welche die aktiven Agenzien in den Gasraum eingesprüht werden.

## Patentansprüche

1. Verfahren zum Reduzieren von übel riechenden Substanzen in Tankbehältern für Schweröl oder verflüssigtes Bitumen, in denen über dem Flüssigkeitsspiegel sich ein Gasraum mit übel riechenden Substanzen befindet, wobei in den Gasraum aktive Agenzien eingesprüht werden, die mindestens 30 Gew.% eines Aldehyds mit mehr als 10 Kohlenstoffatomen im Molekül und einem Siedepunkt von mindestens 150 °C enthalten, welche mit den übel riechenden Substanzen reagieren bzw. diese maskieren und dadurch die Geruchsbelästigung vermindern,
**dadurch gekennzeichnet, dass** die aktiven Agenzien zusammen mit 20 bis 70 Gew.% eines Mineralöls in den Gasraum eingesprüht werden, ohne dass gleichzeitig ein Inertgas eingeleitet wird.

2. Verfahren nach Anspruch 1, **dadurch gekennzeichnet, dass** der Tankbehälter 500 bis 15.000 to Schweröl bzw. Bitumen enthält, wobei der Gasraum über dem Flüssigkeitsspiegel 10 bis 90 % des Gesamtvolumens ausmacht.

3. Verfahren nach Anspruch 1, **dadurch gekennzeichnet, dass** die übel riechenden Substanzen Kohlenwasserstoffe und Schwefelverbindungen enthalten.

4. Verfahren nach Anspruch 1, **dadurch gekennzeichnet, dass** die aktiven Agenzien neben den Aldehyden noch natürlich vorkommende Alkohole, sowie gegebenenfalls Terpene, Ketone und/oder Carbonsäureester, jeweils mit einem Siedepunkt von mindestens 150 °C, enthalten.

## Claims

1. Method for reducing malodorous substances in tank containers for heavy oil or liquefied bitumen, in which above the liquid level there is a gas chamber containing malodorous substances, wherein active agents are sprayed into the gas chamber which contain at least 30 wt.% of an aldehyde with more than 10 carbon atoms in the molecule and a boiling point of at least 150 °C, which react with the malodorous substances or mask the latter and in this way reduce the unpleasant odour, **characterised in that** the active agents are sprayed together with 20 to 70 wt. % of a mineral oil into the gas chamber, without an inert gas being introduced at the same time.

2. Method according to claim 1, **characterised in that** the tank container contains 500 to 15,000 t heavy oil or bitumen, wherein the gas chamber above the liquid level constitutes 10 to 90 % of the total volume.

3. Method according to claim 1, **characterised in that** the malodorous substances contain hydrocarbons and sulphur compounds.

4. Method according to claim 1, **characterised in that** the active agents in addition to the aldehydes also contain naturally occurring alcohols, as well as possibly terpenes, ketones and/or carboxylic acid esters, each with a boiling point of at least 150 °C.

## Revendications

1. Procédé de réduction de substances malodorantes dans des réservoirs pour huiles lourdes ou bitumes liquides dans lesquels se trouve au-dessus du niveau du liquide un espace gazeux comportant des substances malodorantes, dans lequel sont injectés dans l'espace gazeux des agents actifs, contenant au moins 30 % en poids d'un aldéhyde comportant plus de 10 atomes de carbone dans la molécule et présentant un point d'ébullition au moins égal à 150 °C, qui réagissent avec les substances malodorantes ou les masquent et de ce fait réduisent la charge odorante, **caractérisé en ce que** les agents actifs sont injectés dans l'espace gazeux avec 20 à 70 % en poids d'une huile minérale sans qu'un gaz inerte soit introduit simultanément.

2. Procédé selon la revendication 1, **caractérisé en ce que** le réservoir contient 500 à 15 000 tonnes d'huile lourde ou de bitume, l'espace gazeux au-dessus du niveau de liquide formant 10 à 90 % du volume total.

3. Procédé selon la revendication 1, **caractérisé en ce que** les substances malodorantes contiennent des hydrocarbures et des composés soufrés.

4. Procédé selon la revendication 1, **caractérisé en ce que** les agents actifs, en plus des aldéhydes, contiennent encore des alcools naturels, ainsi qu'éventuellement des terpènes, des cétones et/ou des esters d'acide carboxylique, présentant respectivement un point d'ébullition au moins égal à 150 °C.
